# EUROPEAN PATENT APPLICATION

(11) **EP 4 620 554 A1**
(43) Date of publication of application: **24.09.2025**
(21) Application number: 23891491.5
(22) Date of filing: 10.11.2023
(51) Int. Cl.: B01D 39/16, G01N 15/00

(54) **WELL ARRAY FILTER, WELL ARRAY DEVICE, AND METHOD FOR PRODUCING WELL ARRAY FILTER**

(30) Priority: 14.11.2022 JP 2022181804
(71) Applicant: TOKYO OHKA KOGYO CO., LTD., Kawasaki-shi, Kanagawa 211 0012 (JP)
(72) Inventor: SUZUKI Yasuo, Kawasaki-shi, Kanagawa 211-0012 (JP); OHSAKA Takashi, Kawasaki-shi, Kanagawa 211-0012 (JP); MUROTA Atsushi, Kawasaki-shi, Kanagawa 211-0012 (JP); SAKAMAKI Takuji, Kawasaki-shi, Kanagawa 211-0012 (JP); TAKAHASHI Anna, Kawasaki-shi, Kanagawa 211-0012 (JP); SAITO Hirokuni, Kawasaki-shi, Kanagawa 211-0012 (JP); ASAI Takahiro, Kawasaki-shi, Kanagawa 211-0012 (JP)
(74) Representative: Plasseraud IP
(86) International application number: PCT/JP2023/040608
(87) International publication number: WO 2024/106343

(57) **Abstract**

A well array filter (1) is a well array filter including a through-hole (23) of a size through which a subject including at least one of a cell, a particle, and a liquid droplet does not pass in a well bottom (21), a well layer (10) including a plurality of well openings (11) of a size through which the subject is able to pass, and a support layer (20) supporting the well layer (10). The support layer (20) includes a plurality of well bottoms (21) corresponding to the well openings, and at least a part of the support layer (20) between a plurality of neighboring well bottoms (21) is divided.

## Description

### TECHNICAL FIELD

The present invention relates to a well array filter, a well array device, and a well array filter manufacturing method.

Priority is claimed on Japanese Patent Application No. 2022-181804, filed November 14, 2022, the content of which is incorporated herein by reference.

### BACKGROUND ART

For example, an independent well array filter using a stacked film in which a thin film and a support film are stacked is disclosed in Patent Document 1. The thin film includes a first hole portion including one or more holes penetrating the thin film in a thickness direction thereof. The support film includes a second hole portion including one or more holes penetrating the support film in a thickness direction thereof. At least some of the holes of the first hole portion and at least some of the holes of the second hole portion communicate with each other. All openings of the holes of the first hole portion are included in openings of the holes of the second hole portion. For example, a precursive film of the support film is formed by applying a negative photosensitive composition onto the thin film. By performing exposure and developing on the precursive film of the support film, the support film in which holes including openings are regularly arranged is formed on the thin film. The thin film of the stacked film is a connected member.

### Citation List

### Patent Document

Patent Document 1: Japanese Patent No. 6549869

### SUMMARY OF INVENTION

### Technical Problem

However, when a thin film (a support layer) constituting a well array filter is a connected body and has a high aperture ratio, parts in which only the support layer is exposed are enlarged and mechanical strength is likely to decrease in comparison with a case in which the support layer has a low aperture ratio. Accordingly, there is a high likelihood that distortion will occur in the support layer due to a stress caused by curing shrinkage of the support layer. As a result, there is need for providing an independent well array filter with a high aperture ratio.

Therefore, an objective of the present invention is to provide an independent well array filter with a high aperture ratio.

### Solution to Problem

(1) A well array filter according to an aspect of the present invention is a well array filter including: a through-hole of a size through which a subject including at least one of a cell, a particle, and a liquid droplet does not pass in a well bottom; a well layer including a plurality of well openings of a size through which the subject is able to pass; and a support layer supporting the well layer, wherein the support layer includes a plurality of well bottoms corresponding to the well openings, and at least a part of the support layer between a plurality of neighboring well bottoms is divided.

With this configuration, since a stress applied to the support layer (for example, a stress due to curing shrinkage of the support layer) can be absorbed by the divided well bottoms, it is possible to curb distortion of the support layer. Accordingly, it is possible to provide an independent well array filter with a high aperture ratio.

(2) In the well array filter according to (1), the support layer may be completely divided for each well bottom.

The support layer may be partially divided for each well bottom.

With this configuration, since a stress applied to the support layer can be absorbed by the divided well bottoms as a whole, it is possible to more effectively curb distortion of the support layer.

(3) In the well array filter according to (1), the support layer may include a plurality of well bottom structures in which a plurality of well bottoms are connected together and the support layer may be divided for each well bottom structure.

The support layer may include a plurality of well bottom structures in which a plurality of well bottoms are connected together. The support layer may be divided for each well bottom structure.

With this configuration, a stress applied to the support layer can be locally absorbed by the divided well bottoms. In comparison with a case in which the support layer is completely divided for each well bottom, the mechanical strength thereof is higher. For example, when a well array manufactured on a substrate is made into an independent body through lift-off, it is possible to curb a defect such as omission of some of the plurality of divided well bottoms from the independent body.

(4) In the well array filter according to any one of (1) to (3), the well array filter may be formed of a photo-curable resin.

The well array filter may be formed of a photo-curable resin.

With this configuration, it is possible to manufacture a well array filter using photolithography (for example, a photoresist applying step, an exposure step, and a developing step) which is very suitable for micromachining.

(5) In the well array filter according to any one of (1) to (4), an aperture ratio of the well openings may be equal to or greater than 40%, a ratio A/B of a depth A of the well layer to a minimum width B of a partition wall portion between neighboring wells in the well layer may be equal to or greater than 2, and a thickness of the support layer may be equal to or less than 5 µm.

An aperture ratio of the well openings may be equal to or greater than 40%. A ratio A/B of a depth A of the well layer to a minimum width B of a partition wall portion between neighboring wells in the well layer may be equal to or greater than 2. A thickness of the support layer may be equal to or less than 5 µm.

With this configuration, it is easier to increase a density of the well openings in comparison with a case in which the aperture ratio of the well openings is less than 40% and the ratio A/B is less than 2. It is easier to curb autofluorescence in comparison with a case in which the thickness of the support layer is greater than 5 µm.

(6) In the well array filter according to any one of (1) to (5), the support layer may include the through-holes communicating with the well openings.

The support layer may include a recessed portion (a recessed portion recessed to expose the well openings) communicating with the well openings.

With this configuration, it is possible to capture a subject entering the well opening on the support layer and to discharge an object (for example, an object of a smaller size than the subject) other than the subject via the through-hole.

(7) In the well array filter according to any one of (1) to (6), each well opening may have a polygonal shape when seen in a thickness direction of the well layer.

Each well opening may have a polygonal shape when seen in the thickness direction of the well layer.

With this configuration, it is easier to increase the density of the well openings in comparison with a case in which each well opening has a circular shape when seen in the thickness direction of the well layer.

(8) In the well array filter according to any one of (1) to (7), the well layer may include a well wall partitioning the well openings, and the plurality of well bottoms may be divided in a part overlapping a central position in a width direction of the well wall when seen in a thickness direction of the well layer.

The well layer may include a well wall partitioning the well openings. The plurality of well bottoms may be divided in a part overlapping a central position in a width direction of the well wall when seen in a thickness direction of the well layer.

With this configuration, in comparison with a case in which the plurality of well bottoms are divided in a part not overlapping the central position in the width direction of the well wall when seen in the thickness direction of the well layer, the gaps between the divided well bottoms can be reliably filled with the well wall, and thus it is possible to reliably prevent liquid leakage from a part other than the through-hole.

(9) **In** the well array filter according to any one of (1) to (8), a minimum width between the divided well bottoms when seen in a thickness direction of the support layer may be larger than a thickness of the support layer.

With this configuration, in comparison with a case in which the minimum width between the divided well bottoms is equal to or less than the thickness of the support layer, it is easier to divide the well bottoms through photolithography.

(10) **In** the well array filter according to any one of (1) to (9), the well array filter may further include a subject placement surface on which the subject is placed, and the subject placement surface may be covered with a non-cell-adhesive material.

The well array filter may further include a subject placement surface on which the subject is placed. The subject placement surface may be covered with a non-cell-adhesive material.

With this configuration, it is possible to curb adhesion of a cell to the subject placement surface.

The subject placement surface includes a side surface and a top surface of the well wall, a bottom surface of the support layer, and the whole well array filter in addition to a top surface of the well bottom. For example, in view of not leaving a cell on the well wall, it is important to cover the top surface of the well wall with a non-cell-adhesive material.

(11) In the well array filter according to any one of (1) to (10), a line may appear in a part along an outer shape of the well bottoms when a rear surface of the well array filter is observed with an optical microscope or a scanning electron microscope.

With this configuration, when a line appears, a structure of the present invention can be identified, and thus it is possible to contribute to facilitation of infringement identification.

(12) A well array device according to another aspect of the present invention is a well array device for accommodating and arranging the subject and includes the well array filter according to any one of (1) to (11).

The well array device may be a well array device for accommodating and arranging the subject. The well array device may include the well array filter according to any one of (1) to (11).

With this configuration, since the well array filter is provided, it is possible to provide a well array device that can accommodate and arrange more subjects per unit area.

(13) A well array filter manufacturing method according to another aspect of the present invention is a well array filter manufacturing method of manufacturing the well array filter according to any one of (1) to (11), the well array filter manufacturing method including: a first step of forming a sacrificial film which is insoluble in a solvent dissolving materials of the support layer and the well layer on a substrate; a second step of forming the support layer and the well layer on the sacrificial film after the first step; and a third step of dissolving the sacrificial film and separating a structure including the support layer and the well layer from the substrate after the second step.

The well array filter manufacturing method may be a well array filter manufacturing method of manufacturing the well array filter according to any one of (1) to (11). The well array filter manufacturing method may include a first step of forming a sacrificial film which is insoluble in a solvent dissolving materials of the support layer and the well layer on a substrate. The well array filter manufacturing method may include a second step of forming the support layer and the well layer on the sacrificial film after the first step. The well array filter manufacturing method may include a third step of dissolving the sacrificial film and separating a structure including the support layer and the well layer from the substrate after the second step.

With this configuration, since a stress applied to the support layer (for example, a stress due to curing shrinkage of the support layer) can be absorbed by the divided well bottoms in the second step, it is possible to curb distortion of the support layer. Accordingly, it is possible to provide an independent well array filter with a high aperture ratio.

### Advantageous Effects of Invention

With the well array filter, the well array device, and the well array filter manufacturing method according to the aspects, it is possible to provide an independent well array filter with a high aperture ratio.

### BRIEF DESCRIPTION OF DRAWINGS

[FIG. 1] A top view of a well array filter according to a first embodiment.
[FIG. 2] A diagram illustrating a capillary along with a sectional view taken along line II-II in FIG. 1.
[FIG. 3] A top view of a support layer according to the first embodiment.
[FIG. 4] A top view of a well layer according to the first embodiment.
[FIG. 5] A diagram illustrating a well array filter manufacturing method according to the first embodiment., where FIG. 5(A) is a diagram illustrating a first step, FIG. 5(B) is a diagram illustrating a step subsequent to FIG. 5(A), FIG. 5(C) is a diagram illustrating a step subsequent to FIG. 5(B), FIG. 5(D) is a diagram illustrating a step subsequent to FIG. 5(C), FIG. 5(E) is a diagram illustrating a step subsequent to FIG. 5(D), and FIG. 5(F) is a diagram illustrating a step subsequent to FIG. 5(E).
[FIG. 6] A diagram illustrating a well array with a low aperture ratio according to a first comparative example.
[FIG. 7] A diagram illustrating a well array with a high aperture ratio according to a second comparative example.
[FIG. 8] A diagram illustrating a state in which a film is distorted according to the second comparative example.
[FIG. 9] A diagram illustrating operations of the well array filter according to the first embodiment.
[FIG. 10] A top view of a support layer according to a second embodiment.
[FIG. 11] A top view of a well bottom structure according to a third embodiment.
[FIG. 12] A top view of a support layer (before a well layer is formed) on a wafer according to a first example (corresponding to the first embodiment) when the support layer is observed with a scanning electron microscope.
[FIG. 13] A top view of a well layer (after the well layer is formed) on the support layer according to the first example when the well layer is observed with a scanning electron microscope.
[FIG. 14] A top view of a support layer (before a well layer is formed) on a wafer according to a second example (corresponding to the second embodiment) when the support layer is observed with a scanning electron microscope.
[FIG. 15] A top view of a well layer (after the well layer is formed) on the support layer according to the second example when the well layer is observed with a scanning electron microscope.
[FIG. 16] A diagram illustrating a rear surface of a well array filter according to an example (corresponding to a well opening width of 20 µm and a well bottom body width of 25 µm) when the rear surface is observed with an optical microscope, where FIG. 16(A) illustrates observation at 5000 magnification and FIG. 16(B) illustrates observation at 2000 magnification.
[FIG. 17] A diagram illustrating a rear surface of a well array filter according to an example (corresponding to a well opening width of 20 µm) when the rear surface is observed with a scanning electron microscope.
[FIG. 18] A diagram illustrating a rear surface of a well array filter according to an example (corresponding to a well opening width of 50 µm) when the rear surface is observed with a scanning electron microscope.
[FIG. 19] A diagram illustrating dimensions along with a sectional view of a well array filter according to an example when observed with a scanning electron microscope.
[FIG. 20] A diagram illustrating dimensions along with a top view of a well.
[FIG. 21] A diagram illustrating a well array filter according to Example 1 when the well array filter is observed with a scanning electron microscope, where FIG. 21(A) is a perspective view, FIG. 21(B) is a sectional view, and FIG. 21(C) is a top view.
[FIG. 22] A diagram illustrating a well array filter according to Example 2 when the well array filter is observed with a scanning electron microscope, where FIG. 22(A) is a perspective view, FIG. 22(B) is a sectional view, and FIG. 22(C) is a top view.
[FIG. 23] A diagram illustrating a well array filter according to Example 3 when the well array filter is observed with a scanning electron microscope, where FIG. 23(A) is a perspective view, FIG. 23(B) is a sectional view, and FIG. 23(C) is a top view.
[FIG. 24] A diagram illustrating a well array filter according to Example 4 when the well array filter is observed with a scanning electron microscope, where FIG. 24(A) is a perspective view, FIG. 24(B) is a sectional view, and FIG. 24(C) is a top view.
[FIG. 25] A diagram illustrating a well array filter according to Example 5 when the well array filter is observed with a scanning electron microscope, where FIG. 25(A) is a perspective view, FIG. 25(B) is a sectional view, and FIG. 25(C) is a top view.
[FIG. 26] A diagram illustrating a well array filter according to Example 6 when the well array filter is observed with a scanning electron microscope, where FIG. 26(A) is a perspective view, FIG. 26(B) is a sectional view, and FIG. 26(C) is a top view.
[FIG. 27] A diagram illustrating a well array filter according to Example 7 when the well array filter is observed with a scanning electron microscope, where FIG. 27(A) is a perspective view, FIG. 27(B) is a sectional view, and FIG. 27(C) is a top view.
[FIG. 28] A diagram illustrating a well array filter according to Example 8 when the well array filter is observed with a scanning electron microscope, where FIG. 28(A) is a perspective view, FIG. 28(B) is a sectional view, and FIG. 28(C) is a top view.
[FIG. 29] A top view of a well array filter according to a comparative example.
[FIG. 30] A top view of a support layer according to a comparative example.
[FIG. 31] A top view of a well layer according to a comparative example.
[FIG. 32] A diagram illustrating a top surface of a well array filter according to a comparative example when the well array filter is observed with an optical microscope.
[FIG. 33] A diagram illustrating a rear surface of a well array filter according to a comparative example when the well array filter is observed with a scanning electron microscope.
[FIG. 34] A diagram illustrating a rear surface of a well array filter according to a comparative example when the well array filter is observed with a scanning electron microscope.

### DESCRIPTION OF EMBODIMENTS

Hereinafter, embodiments of the present invention will be described with reference to the accompanying drawings. In the drawings, an XYZ coordinate system is illustrated according to necessity. In this specification, directions are defined on the basis of the XYZ coordinate system according to necessity. In embodiments, an X direction is an example of a first direction which is parallel to a horizontal plane. A Y direction is an example of a second direction which is perpendicular to the first direction in the horizontal plane. A Z direction is an example of a third direction which is perpendicular to the first direction and the second direction and which is parallel to a vertical direction. The +Z direction corresponds to an upper side in the vertical direction. The -Z direction corresponds to a lower side in the vertical direction. The drawings used for the following description may enlarge featured parts for the purpose of easy understanding of features of the present invention. In the drawings used for the following description, dimensions, ratios, and the like of constituents may not be the same as actual ones.

### <First embodiment>

### <Well array filter>

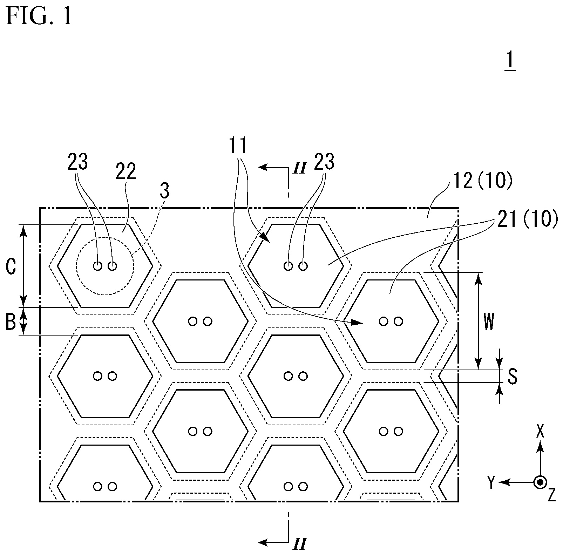

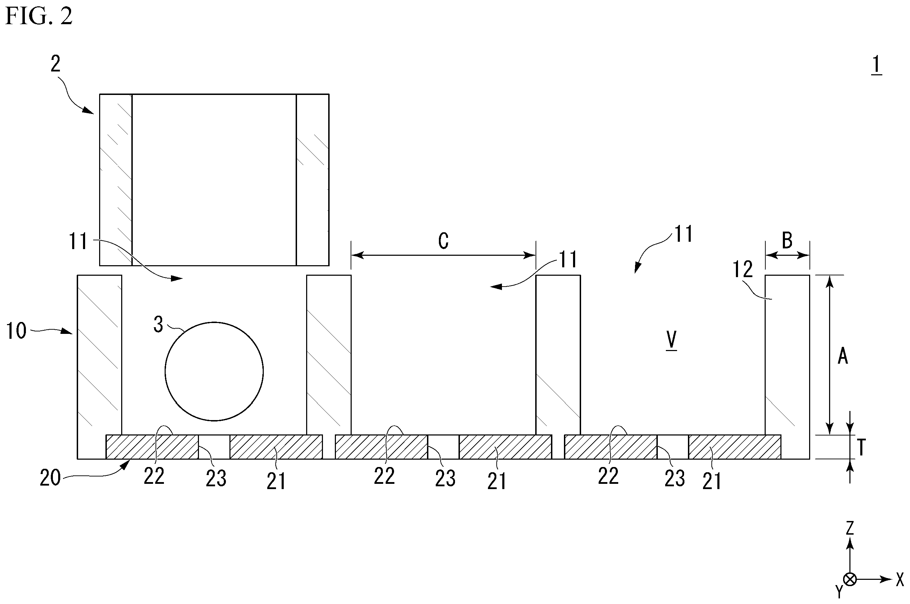

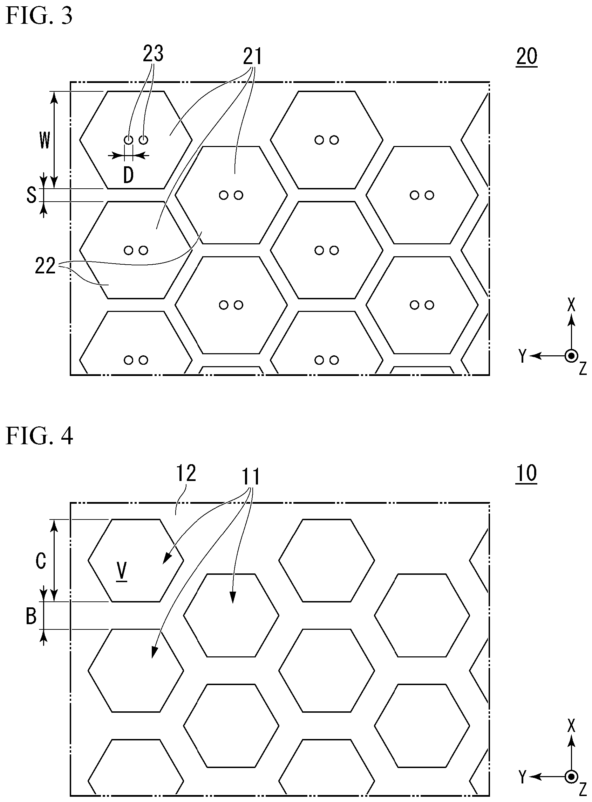

FIG. 1 is a top view of a well array filter 1 according to a first embodiment. FIG. 2 is a diagram illustrating a capillary 2 along with a sectional view taken along line II-II in FIG. 1. FIG. 3 is a top view of a support layer 20 according to the first embodiment. FIG. 4 is a top view of a well layer 10 according to the first embodiment.

As illustrated in FIGS. 1 to 4, a well array filter 1 includes a through-hole 23 of a size through which a subject 3 including at least one of a cell, a particle, and a liquid droplet does not pass in a well bottom 21.

The well array filter 1 is a constituent of a well array device for accommodating and arranging a subject 3 (cells, particles, or liquid droplets). For example, the well array device may include a sample chamber that can individually accommodate samples (cells, particles, or liquid droplets) as the subject 3. In this case, the well array filter 1 may be a film-like member including accommodation portions corresponding to the sample chambers (well bottoms 21 corresponding to well openings 11).

The well array filter 1 includes a well layer 10 including a plurality of well openings 11 of a size through which the subject 3 is able to pass and a support layer 20 supporting the well layer 10. The support layer 20 includes a plurality of well bottoms 21 corresponding to the well openings 11. At least a part between a plurality of well bottoms 21 which are adjacent to each other in the support layer 20 are divided. The support layer 20 according to the present embodiment is completely divided for each well bottom 21.

The support layer 20 may be partially divided for each well bottom 21.

For example, the well array filter 1 has flexibility with which it is not broken even when it is pressed with a capillary 2. For example, the well array filter 1 can have flexibility with which the well layer 10 and the support layer 20 are not broken even when the well layer 10 on the support layer 20 is pressed with the capillary 2 from above.

For example, the well array filter 1 is preferably formed to put a focus on the whole film surface even when a focal distance is fixed.

The well array filter 1 according to the present embodiment is formed of a photo-curable resin. For example, the well array filter 1 is formed of a negative resist. The material of the well array filter 1 can be arbitrary according to the subject 3 accommodated therein. When through-holes 23 of a size through which the subject 3 cannot pass are regularly arranged, the material of the well array filter 1 is preferably a negative photoresist or a poly dimethyl siloxane (PDMS) and most preferably a negative photoresist.

The material of the well array filter 1 can additionally include one or more materials selected from a group consisting of a negative photoresist, a poly dimethyl siloxane (PDMS), and mixtures thereof and preferably includes a negative photoresist.

The well array filter 1 includes a subject placement surface 22 on which the subject 3 is placed. The subject placement surface 22 includes a side surface and a top surface of a well wall 12, a bottom surface of the support layer 20, and the whole well array filter 1 in addition to the top surface of the well bottom 21. For example, in view of not leaving a cell on the well wall 12, it is important to cover the top surface of the well wall 12 with a non-cell-adhesive material. In the present embodiment, the whole of the well array filter 1 (corresponding to the subject placement surface 22) is covered with a non-cell-adhesive material. For example, the subject placement surface 22 is coated with a non-cell-adhesive polymer. Examples of the non-cell-adhesive polymer include 2-methacryloxy ethyl phosphorylcholine (MPC) polymer, polyoligoehtylene glycol methacrylate (PEG), poly-2-methoxy ethyl acrylate (PMEA), and polyvinyl alcohol (PVA).

An aperture ratio of the well openings 11 according to the present embodiment is equal to or greater than 40%. The aperture ratio of the well openings 11 (hereinafter referred to as a "well opening ratio") corresponds to a ratio of the total area of the well openings 11 to the total area of the well array filter 1 when seen from the top view of the well array filter 1 (in a plan view) (when seen in the thickness direction of the well layer 10). For example, in view of realizing the well array filter 1 with a high aperture ratio, the well opening ratio is preferably equal to or greater than 50% and more preferably equal to or greater than 60%.

Each well opening 11 according to the present embodiment has a hexagonal shape (an example of a polygonal shape) in a plan view. Specifically, a shape in a plan view of a reticle (an example of a photomask) for forming the well opening 11 is a hexagonal shape. On the other hand, the shape in a plan view of the well opening 11 is actually a hexagonal shape including rounded corners. The shapes in a plan view of a plurality of well openings 11 are substantially the same hexagonal outer shape.

Each well opening 11 may include a straight edge when seen in the thickness direction of the well layer 10.

For example, a width of the well opening 11 (hereinafter also referred to as a "well opening width") C is preferably a size which one or more subjects 3 (cells, particles, or liquid droplets) can enter. When the shape in a plan view of the well opening 11 is hexagonal, the well opening width C corresponds to a gap between two opposite sides in the well opening 11. In the illustrated example, the well opening width C has a size which one spherical cell or particle enters as the subject 3.

The well layer 10 includes a well wall 12 for partitioning the well openings 11. A ratio A/B of a depth A of the well layer 10 to a minimum width B of a partition wall portion between neighboring wells in the well layer 10 is equal to or greater than 2. The depth of the well layer 10 (hereinafter also referred to as a "well depth") A corresponds to a distance from the top surface of the support layer 20 to the top surface of the well layer 10. When the shape in a plan view of the well opening 11 is hexagonal, the minimum width of the partition wall portion between neighboring wells in the well layer 10 (hereinafter also referred to as a "well wall width") B corresponds to a gap between two opposite sides in the well layer 10 (the well wall 12 defining the well openings 11). For example, in view of further increasing the density of the well openings 11, the ratio A/B is preferably equal to or greater than 2.5 and more preferably equal to or greater than 4.

For example, the well depth A is preferably larger than a size of a cell or a particle. For example, the well depth A is preferably equal to or greater than 10 µm. For example, when the diameter of a spherical cell or particle is about 15 µm, the well depth A is preferably equal to or greater than 20 µm and more preferably equal to or greater than 30 µm in view of more reliably accommodating the cell or particle. For example, in view of not hindering observation and curbing autofluorescence, the well depth A is preferably equal to or less than 200 µm.

For example, the well wall width B is preferably a size with which a cell or particle is not left on the well wall 12. For example, the well wall width B is preferably less than the size of the cell or particle. For example, the well wall width B is preferably equal to or less than 20 µm. For example, when the diameter of the spherical cell or particle is about 15 µm, the well wall width B is preferably equal to or less than 15 µm and more preferably equal to or less than 10 µm in view of not more reliably leaving a cell on the well wall 12 and further increasing the density of the well openings 11. The well wall width B in the present embodiment is about 10 µm.

A well volume V is calculated on the basis of the area in a plan view of the well opening 11 and the well depth A. For example, when contents of a well is recovered and used for subsequent analysis using a capillary, the well volume V is preferably a volume which can minimize an amount of liquid for the subsequent analysis.

For example, a thickness T of the support layer 20 is preferably a thickness which can reduce autofluorescence as much as possible. The thickness T of the support layer 20 corresponds to a gap (an average value) between the top surface and the bottom surface of the support layer 20. For example, the thickness T of the support layer 20 is equal to or less than 5 µm. For example, in view of further reducing autofluorescence, the thickness T of the support layer 20 is more preferably equal to or less than 3 µm.

The support layer 20 includes through-holes 23 communicating with the corresponding well openings 11. In the illustrated example, the support layer 20 includes two through-holes 23 which are circular in a plan view at the center of the well bottom 21 corresponding to each well opening 11. The configuration of the through-holes 23 is not particularly limited and may be any shape in a plan view as long as it communicates with the well opening 11, and the number of through-holes 23 is also not limited.

The support layer 20 may include a recessed portion (a recessed portion recessed to expose the well opening 11) communicating with the well opening 11.

For example, the diameter of each through-hole 23 is preferably is a size through which the subject 3 (cells, particles, or liquid droplets) cannot pass. For example, the diameter of the through-hole 23 (hereinafter also referred to as a "through-hole diameter") D is preferably is a size which can discharge an object other than the subject 3 (for example, an object having a smaller size than the subject 3). For example, when the diameter of the spherical cell or particle is about 15 µm, the through-hole diameter D is preferably equal to or less than 10 µm and more preferably equal to or less than 5 µm in view of more reliably allowing the cell or particle not to pass through the through-hole 23.

A plurality of well bottoms 21 according to the present embodiment are divided in parts overlapping the central positions in the width direction of the well wall 12 in a plan view (corresponding to the center of the well wall width B). In the well array filter 1 according to the present embodiment, in a pan view (when seen in the thickness direction of the support layer 20), a minimum width between the divided well bottoms 21 (hereinafter also referred to as a "well inter-bottom width") S is larger than the thickness T of the support layer 20. In the present embodiment, the well inter-bottom width S is smaller than the well wall width B. In the drawing, reference sign W denotes a width of the well bottom 21 in a plan view (hereinafter also referred to as a "well bottom body width").

### <Method of manufacturing well array filter>

A method of manufacturing the well array filter 1 according to the present embodiment is a method of manufacturing the well array filter 1 and includes a first step of forming a sacrificial film 31 which is insoluble in a solvent dissolving the materials of the support layer 20 and the well layer 10 on a substrate 30, a second step of forming the support layer 20 and the well layer 10 on the sacrificial film 31 after the first step, and a third step of dissolving the sacrificial film 31 and separating a structure including the support layer 20 and the well layer 10 from the substrate 30 after the second step.

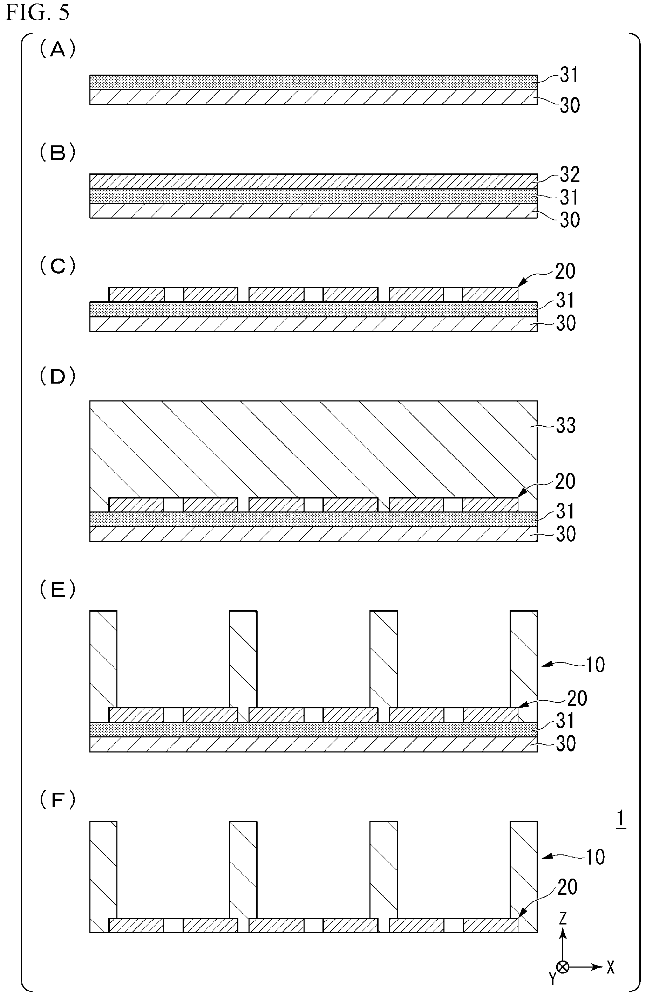

FIG. 5 is a diagram illustrating the well array filter 1 manufacturing method according to the first embodiment. FIG. 5(A) is a diagram illustrating a first step, FIG. 5(B) is a diagram illustrating a step subsequent to FIG. 5(A), FIG. 5(C) is a diagram illustrating a step subsequent to FIG. 5(B), FIG. 5(D) is a diagram illustrating a step subsequent to FIG. 5(C), FIG. 5(E) is a diagram illustrating a step subsequent to FIG. 5(D), and FIG. 5(F) is a diagram illustrating a step subsequent to FIG. 5(E). Referring to FIG. 5(A) together, in the first step, the sacrificial film 31 is formed on the substrate 30.

Examples of the sacrificial film 31 include a polyvinyl alcohol resin, dextrin, dextran, gelatin, glue, casein, shellac, gum arabic, starch, protein, polyacrylamide, sodium polyacrylate, polyvinylmethyl ether, styrene-based elastomer, a copolymer of methylvinyl ether and maleic anhydride, a copolymer of vinyl acetate and itaconate, polyvinylpyrrollidone, acetyl cellulose, hydroxyl ethyl cellulose, and sodium alginate. Examples of the substrate 30 include silicon, glass, and a PET film.

The method of forming the sacrificial film 31 is not particularly limited and is preferably a method of applying a coating solution for forming the sacrificial film 31 onto the substrate 30. Examples of the method of applying the coating solution for forming the sacrificial film 31 onto the substrate 30 include methods using a contact-transfer coater such as a roll coater, a reverse coater, and a bar coater or a non-contact coater such as a spinner (a spin coater) and a curtain flow coater. By drying a coated film formed through coating using a method such as heating, the sacrificial film 31 is formed. The thickness of the sacrificial film 31 is not particularly limited. For example, in view of rapidly dissolving the sacrificial film 31, the thickness of the sacrificial film 31 is preferably equal to or greater than 10 nm and equal to or less than 180 nm. After the first step, the second step is subsequently performed.

Referring to FIGS. 5(B) to 5(E) together, in the second step, the support layer 20 and the well layer 10 are formed on the sacrificial film 31.

By applying a resist of the support layer 20 onto the sacrificial film 31, a precursive film 32 of the support layer 20 is formed (see FIG. 5(B)). For example, the thickness of the precursive film 32 of the support layer 20 is adjusted such that the support layer 20 is formed in a thickness which is equal to or greater than 1.0 µm and equal to or less than 1.2 µm. For example, the material of the resist of the support layer 20 is a negative photoresist.

Then, the surface of the precursive film 32 of the support layer 20 is exposed selectively in position and then developed. For example, the method of exposing the surface of the precursive film 32 of the support layer 20 selectively in position is a method of performing exposure via a mask for a negative photoresist. For example, a developer is selected according to the type of the negative photoresist. Through this method, the support layer 20 is formed on the sacrificial film 31 (see FIG. 5(C)).

Then, by applying a resist of the well layer 10 onto the sacrificial film 31, a precursive film 33 of the well layer 10 is formed (see FIG. 5(D)). For example, the thickness of the precursive film 33 of the well layer 10 is adjusted such that the well layer 10 is formed in a thickness which is equal to or greater than 23 µm and equal to or less than 25 µm. For example, the material of the resist of the well layer 10 is a negative photoresist.

Then, the surface of the precursive film 33 of the well layer 10 is exposed selectively in position and then developed. For example, the method of exposing the surface of the precursive film 33 of the well layer 10 selectively in position is a method of performing exposure via a mask for a negative photoresist. For example, a developer is selected according to the type of the negative photoresist. Through this method, the support layer 20 and the well layer 10 are formed on the sacrificial film 31 (see FIG. 5(E)). After the second step, the third step is subsequently performed.

Referring to FIG. 5(F) together, in the third step, the sacrificial film 31 is dissolved, and the structure including the support layer 20 and the well layer 10 is separated from the substrate 30. A solution for dissolving the sacrificial film 31 is not particularly limited as long as it is a liquid which does not deteriorate or dissolve the support layer 20 and the well layer 10. Examples of the solution for dissolving the sacrificial film 31 include water, an acidic or alkali aqueous solution, an organic solvent, and an aqueous solution of an organic solvent.

Through these steps, the well array filter 1 according to the present embodiment is manufactured. The well array filter 1 manufactured in this way, at least a part between a plurality of well bottoms 21 adjacent to each other in the support layer 20 is divided. Accordingly, the well array filter 1 can be suitably used as an independent well array filter with a high aperture ratio.

When the support layer 20 is a connected body, there is a following problem.

First, a case in which the support layer is a connected body and the aperture ratio is low will be described as a first comparative example.

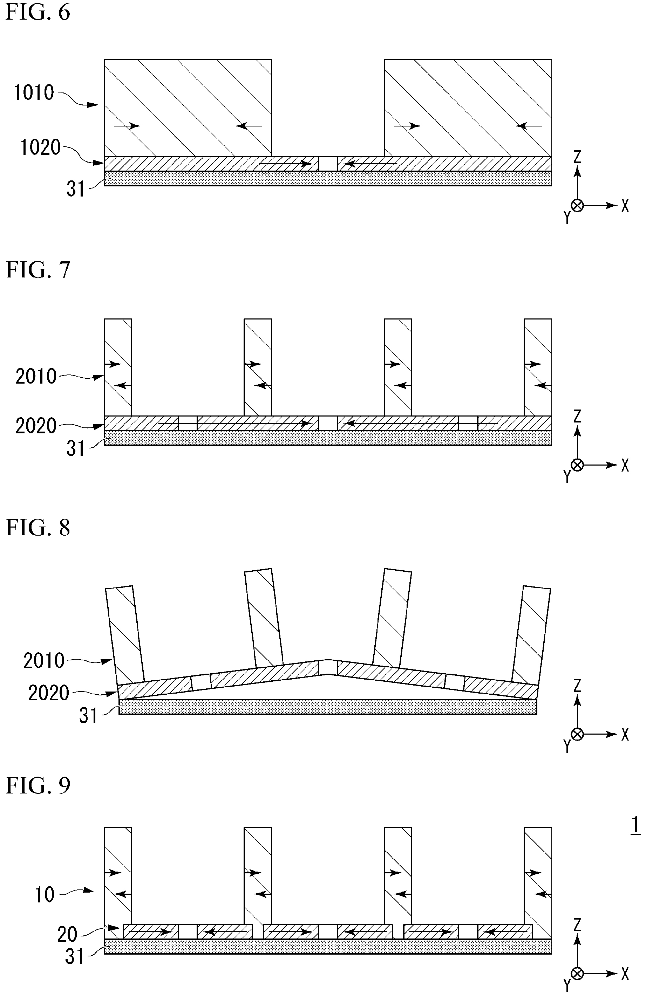

FIG. 6 is a diagram illustrating a well array with a low aperture ratio according to the first comparative example.

As illustrated in FIG. 6, a well array with a low aperture ratio according to the first comparative example includes a support layer 1020 formed to be continuous on the sacrificial film 31 (hereinafter also referred to as a "continuous support layer 1020") and a well layer 1010 formed on the continuous support layer 1020. The well opening ratio in the first comparative example is lower than the well opening ratio in the present embodiment.

**In** the first comparative example, a part of a stress due to curing shrinkage of the continuous support layer 1020 under the well layer 1010 is cancelled by a stress due to curing shrinkage of the well layer 1010. In the first comparative example, the part of the well layer 1010 has a large thickness and thus high mechanical strength, and shrinkage is less likely to occur. Since a part in which only the support layer 1020 is exposed is small and is surrounded with the well layer 1010 with a large thickness and high mechanical strength, the support layer 1020 is less distorted due to curing shrinkage.

A case in which the support layer is a connected body and the aperture ratio is high will be described below as a second comparative example.

FIG. 7 is a diagram illustrating a well array with a high aperture ratio according to the second comparative example. FIG. 8 is a diagram illustrating a state in which a film is distorted in the second comparative example.

Referring to FIGS. 7 and 8 together, a well array with a high aperture ratio according to the second comparative example includes a continuous support layer 2020 formed on the sacrificial film 31 and a well layer 2010 formed on the continuous support layer 2020. The well opening ratio in the second comparative example is higher than the well opening ratio in the first comparative example.

In the second comparative example, since a proportion occupied by the part of the well layer 2010 is less than that in the first comparative example, a stress due to curing shrinkage of the continuous support layer 2020 is less likely to be cancelled by a stress due to curing shrinkage of the well layer 2010. Accordingly, in the second comparative example, the part of the continuous support layer 2020 has low mechanical strength and is likely to shrink. For example, when the stress is concentrated on a part of the continuous support layer 2020, there is a high likelihood of a distorted state as illustrated in FIG. 8.

On the other hand, in the well array filter 1 according to the present embodiment, since at least a part between a plurality of well bottoms 21 adjacent to each other in the support layer 20 is divided, there is a low likelihood that the aforementioned problem will occur.

FIG. 9 is a diagram illustrating operations of the well array filter 1 according to the first embodiment.

As illustrated in FIG. 9, the well array filter 1 according to the present embodiment has a structure in which the support layer 20 is fragmented in a tiles shape or a structure in which tiles are partially connected. In the illustrated example, tiles (corresponding to the well bottoms 21) of the support layer 20 are completely divided.

In the present embodiment, curing shrinkage of the support layer 20 is caused locally, but not in the whole layer. Accordingly, in the present embodiment, the stress due to curing shrinkage of the support layer 20 decreases more than in the comparative examples. As a result, according to the present embodiment, it is possible to manufacture an independent well array filter 1 with a high aperture ratio using the support layer 20 with a less thickness than in the comparative examples.

### <Operations and advantages>

As described above, the well array filter 1 according to the present embodiment includes the through-holes 23 of a size through which a subject 3 including at least one of a cell, a particle, and a liquid droplet does not pass in the well bottoms 21. The well array filter 1 includes the well layer 10 including a plurality of well openings 11 of a size through which the subject 3 is able to pass and the support layer 20 supporting the well layer 10. The support layer 20 includes a plurality of well bottoms 21 corresponding to the well openings 11. At least a part between a plurality of well bottoms 21 adjacent to each other in the support layer 20 is divided.

With this configuration, since a stress applied to the support layer 20 (for example, a stress due to curing shrinkage of the support layer 20) can be absorbed by the divided well bottoms 21, it is possible to curb distortion of the support layer 20. Accordingly, it is possible to provide an independent well array filter 1 with a high aperture ratio.

The support layer 20 according to the present embodiment is completely divided for each well bottom 21.

With this configuration, since a stress applied to the support layer 20 can be absorbed by the divided well bottoms 21 as a whole, it is possible to more effectively curb distortion of the support layer 20.

The well array filter 1 according to the present embodiment is formed of a photo-curable resin.

With this configuration, it is possible to manufacture a well array filter 1 using photolithography (for example, a photoresist applying step, an exposure step, and a developing step) which is very suitable for micromachining.

The aperture ratio of the well openings 11 according to the present embodiment is equal to or greater than 40%. The ratio A/B of the depth A of the well layer 10 to the minimum width B of the partition wall portion between neighboring wells in the well layer 10 is equal to or greater than 2. The thickness T of the support layer 20 is equal to or less than 5 µm.

With this configuration, it is easier to increase the density of the well openings 11 in comparison with a case in which the aperture ratio of the well openings 11 is less than 40% and the ratio A/B is less than 2. It is easier to curb autofluorescence in comparison with a case in which the thickness T of the support layer 20 is greater than 5 µm.

The support layer 20 according to the present embodiment includes the through-holes 23 communicating with the well openings 11.

With this configuration, it is possible to capture a subject 3 entering the well opening 11 on the support layer 20 and to discharge an object (for example, an object of a smaller size than the subject 3) other than the subject 3 via the through-hole 23.

Each well opening 11 according to the present embodiment is polygonal in a plan view.

With this configuration, it is easier to increase the density of the well openings 11 in comparison with a case in which each well opening 11 is circular in a plan view.

The well layer 10 according to the present embodiment includes the well wall 12 partitioning the well openings 11. The plurality of well bottoms 21 are divided in a part overlapping the central position in the width direction of the well wall 12 in a plan view.

With this configuration, in comparison with a case in which the plurality of well bottoms 21 are divided in a part not overlapping the central position in the width direction of the well wall 12 in a plan view, a gap between the divided well bottoms 21 can be reliably covered with the well wall 12, and thus it is possible to reliably prevent liquid leakage from a part other than the through-holes 23.

In the well array filter 1 according to the present embodiment, the minimum width S between the divided well bottoms 21 in a plan view is larger than the thickness T of the support layer 20.

With this configuration, in comparison with a case in which the minimum width S between the divided well bottoms 21 is equal to or less than the thickness T of the support layer 20, it is easier to divide the well bottoms 21 through photolithography.

The well array filter 1 according to the present embodiment includes the subject placement surface 22 on which the subject 3 is placed. The subject placement surface 22 is covered with a non-cell-adhesive material.

With this configuration, it is possible to curb adhesion of a cell to the subject placement surface 22. The subject placement surface 22 includes the side surface and the top surface of a well wall 12, the bottom surface of the support layer 20, and the whole well array filter 1 in addition to the top surface of the well bottoms 21. In the present embodiment, the whole well array filter 1 (including the top surface of the well wall 12 is covered with a non-cell-adhesive material. Accordingly, the configuration according to the present embodiment is suitable in view of not leaving a cell on the well wall 12.

The well array device according to the present embodiment is a well array device for accommodating and arranging the subject 3 and includes the aforementioned well array filter 1.

With this configuration, since the well array filter 1 is provided, it is possible to provide a well array device that can accommodate and arrange more subjects per unit area.

The well array device may be a well array device for accommodating and arranging the subject 3. The well array device may include the aforementioned well array filter 1.

The well array filter 1 manufacturing method according to the present embodiment is a method of manufacturing the well array filter 1 and includes a first step of forming a sacrificial film 31 which is insoluble in a solvent dissolving materials of the support layer 20 and the well layer 10 on a substrate 30, a second step of forming the support layer 20 and the well layer 10 on the sacrificial film 31 after the first step, and a third step of dissolving the sacrificial film 31 and separating a structure including the support layer 20 and the well layer 10 from the substrate 30 after the second step.

With this method, since a stress applied to the support layer 20 (for example, a stress due to curing shrinkage of the support layer 20) can be absorbed by the divided well bottoms 21 in the second step, it is possible to curb distortion of the support layer 20. Accordingly, it is possible to provide an independent well array filter 1 with a high aperture ratio.

The well array filter 1 manufacturing method may be a method of manufacturing the well array filter 1. The well array filter 1 manufacturing method may include the first step of forming the sacrificial film 31 which is insoluble in a solvent dissolving materials of the support layer 20 and the well layer 10 on the substrate 30. The well array filter 1 manufacturing method may include the second step of forming the support layer 20 and the well layer 10 on the sacrificial film 31 after the first step. The well array filter 1 manufacturing method may include the third step of dissolving the sacrificial film 31 and separating a structure including the support layer 20 and the well layer 10 from the substrate 30 after the second step.

### <Second embodiment>

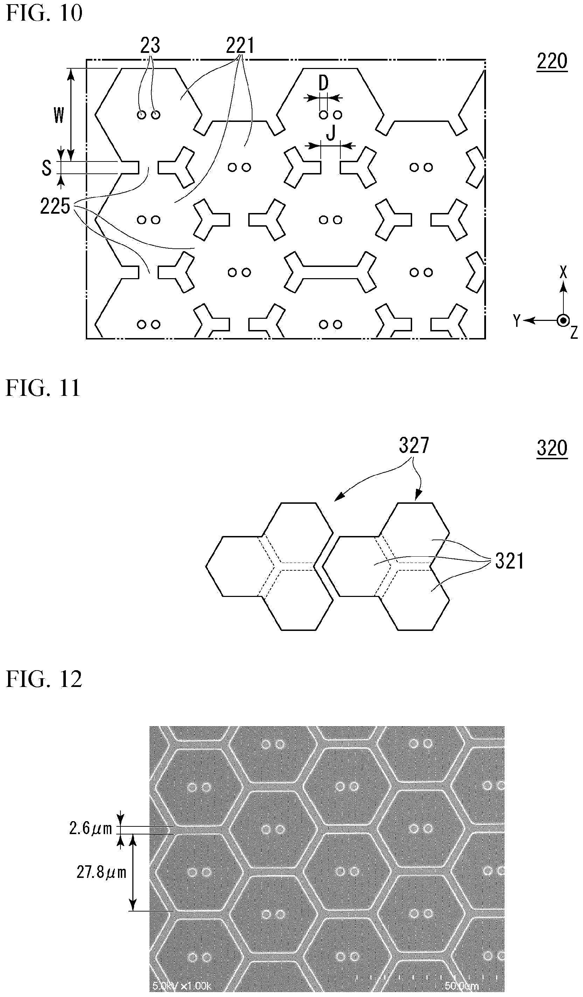

FIG. 10 is a top view of a support layer 220 according to a second embodiment.

In the first embodiment, an example in which the support layer 20 is completely divided for each well bottom 21 has been described above, and the present invention is not limited thereto. For example, as illustrated in FIG. 10, the support layer 220 may not be completely divided or each well button 221. In the second embodiment, the same constituents as in the first embodiment will be referred to by the same reference signs, and detailed description thereof will be omitted.

The support layer 220 according to the present embodiment includes a plurality of well bottoms 221 and connecting portions 225 connecting two neighboring well bottoms 221. In the present embodiment, the support layer 220 is partially divided for each well bottom 221. In the drawing, reference sign J denotes a width of the connecting portion 225 in a plan view (hereinafter also referred to as a "connecting portion width").

In the illustrated example, the shape in a plan view of each well bottom 221 is a hexagonal shape (an example of a polygonal shape). The connecting portion 225 is provided to connect central parts of two opposite sides between two neighboring hexagonal shapes in a plan view. The installation positions of the connecting portions 225 are not limited thereto. For example, the connecting portions 225 may be provided to connect corners of two opposite sides between two neighboring hexagonal shapes in a plan view.

### <Operations and advantages>

As described above, the support layer 220 according to the present embodiment is partially divided for each well bottom 221.

With this configuration, a stress applied to the support layer 220 can be locally absorbed by the divided well bottoms 221. In comparison with a case in which the support layer 20 is completely divided for each well bottom 21, the mechanical strength thereof is higher. For example, at the time of lift-off of lithography, it is possible to curb a defect such as omission of the well bottoms 221.

### <Third embodiment>

FIG. 11 is a top view of a well bottom structure 327 according to a third embodiment.

In the first embodiment, an example in which the support layer 20 is completely divided for each well bottom 21 has been described above, and the present invention is not limited thereto. For example, as illustrated in FIG. 11, a support layer 320 may include a plurality of well bottom structures 327 in which a plurality of well bottoms 321 are connected together. The through-holes 23 and the like are not illustrated in FIG. 11. In the third embodiment, detailed description of the same constituents as in the first embodiment will be omitted.

The support layer 320 according to the present embodiment is divided for each well bottom structure 327. In the illustrated example, the well bottom structure 327 has a configuration in which three well bottoms 321 are connected together. The configuration of the well bottom structure 327 is not limited thereto. For example, the well bottom structure 327 may have a configuration in which two or four or more well bottoms 321 are connected together.

### <Operations and advantages>

As described above, the support layer 320 according to the present embodiment includes a plurality of well bottom structures 327 in which a plurality of well bottoms 321 are connected together. The support layer 320 is divided for each well bottom structure 327.

With this configuration, a stress applied to the support layer 320 can be locally absorbed by the divided well bottoms 321. In comparison with a case in which the support layer 20 is completely divided for each well bottom 21, the mechanical strength thereof is higher. For example, at the time of lift-off of lithography, it is possible to curb a defect such as omission of the well bottom 321.

### <Modified examples>

In the aforementioned embodiments, an example in which the well array filter is formed of a photo-curable resin has been described above, and the present invention is not limited thereto. For example, the well array filter may be formed of a thermosetting resin. For example, the material of the well array filter can be changed according to design specifications.

In the aforementioned embodiment, an example in which the aperture ratio of the well openings is equal to or greater than 40% and the ratio A/B of the depth A of the well layer to the minimum width B of the partition wall portion between neighboring wells in the well layer is equal to or greater than 2 and the thickness of the support layer is equal to or less than 5 µm has been described above, and the present invention is not limited thereto. For example, the aperture ratio of the well openings may be less than 40%. For example, the ratio A/B may be less than 2. For example, the thickness of the support layer may be greater than 5 µm. For example, the aperture ratio of the well openings, the ratio A/B, and the thickness of the support layer can be changed according to design specifications..

In the aforementioned embodiments, an example in which the support layer includes the through-hole communicating with the well opening has been described above, and the present invention is not limited thereto. For example, the support layer may not include a through-hole communicating with a well opening. For example, the configuration of the support layer can be changed according to design specifications.

In the aforementioned embodiments, an example in which the well opening has a polygonal shape when seen in the thickness direction of the well layer has been described above, and the present invention is not limited thereto. For example, the well opening may have a circular shape when seen in the thickness direction of the well layer. For example, the shape of the well opening when seen in the thickness of the well layer (corresponding to the shape in a plan view) can be changed according to design specifications.

In the aforementioned embodiments, an example in which the well layer includes a well wall defining the well openings and a plurality of well bottoms are divided in parts overlapping the center positions in the width direction of the well wall when seen in the thickness direction of the well layer has been described above, and the present invention is not limited thereto. For example, a plurality of well bottoms may be divided in parts not overlapping the center positions in the width direction of the well wall when seen in the thickness direction of the well layer. For example, the configuration of the well bottoms can be changed according to design specifications.

In the aforementioned embodiments, an example in which the minimum width between the divided well bottoms when seen in the thickness direction of the support layer is larger than the thickness of the support layer has been described above, and the present invention is not limited thereto. For example, the minimum width between the divided well bottoms may be equal to or less than the thickness of the support layer. For example, the magnitude of the minimum width between the divided well bottoms when seen in the thickness direction of the support layer can be changed according to design specifications.

In the aforementioned embodiments, an example in which the well array filter includes the subject placement surface on which a subject is placed and the subject placement surface is covered with a non-cell-adhesive material has been described above, and the present invention is not limited thereto. For example, the subject placement surface may not be covered with the non-cell-adhesive material. For example, at least a part of the subject placement surface may be exposed to the outside. For example, the subject placement surface may be covered with a cell-adhesive material. For example, coating of the subject placement surface can be changed according to design specifications.

In the present embodiments, samples (cells or particles) which are accommodated and arranged by the well array filter are not particularly limited. For example, when cells are used as the sample, the sample may include only a single cell or a group of a plurality of cells. For example, a cell includes a cell mass (a cell aggregation).

On the other hand, the constituents in the aforementioned embodiments can be replaced with known constituents without departing from the gist of the present invention. The aforementioned modified examples may be combined.

### Examples

The well array filter according to the aforementioned embodiments of the present invention will be described below in detail in conjunction with examples. The following examples are specific examples to which the present invention is applied and do not limit the present invention.

### <Observation result of first example (corresponding to first embodiment)>

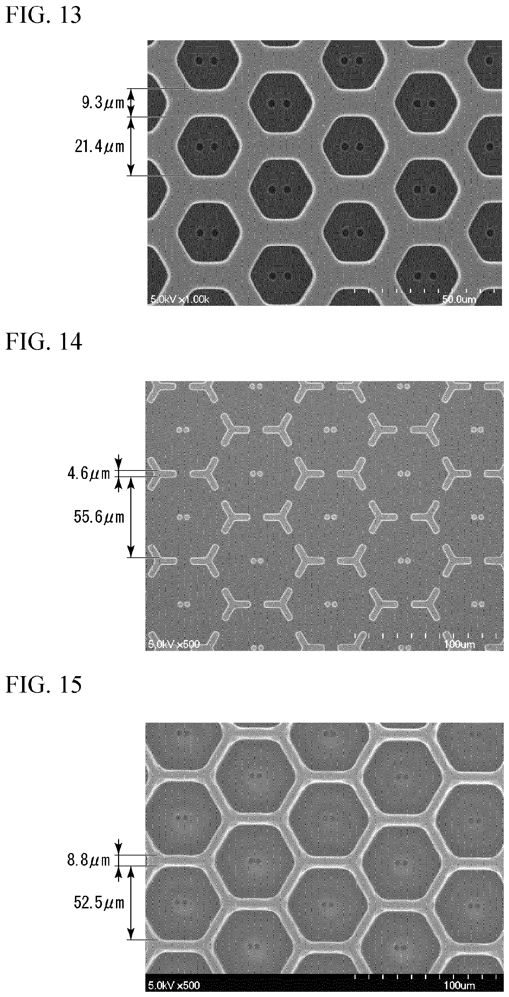

FIG. 12 is a top view of a support layer (before a well layer is formed) on a wafer according to a first example (corresponding to the first embodiment) when the support layer is observed with a scanning electron microscope. FIG. 13 is a top view of a well layer (after the well layer is formed) on the support layer according to the first example when the well layer is observed with a scanning electron microscope.

Referring to FIGS. 12 and 13 together, in the first example, a well array filter was manufactured under the condition in which the well inter-bottom width (2.6 µm) is smaller than the well wall width (9.3 µm). In the first example, the well bottom body width was 27.8 µm, and the well opening width was 21.4 µm.

### <Observation result of second example (corresponding to second embodiment)>

FIG. 14 is a top view of a support layer (before a well layer is formed) on a wafer according to a second example (corresponding to the second embodiment) when the support layer is observed with a scanning electron microscope. FIG. 15 is a top view of a well layer (after the well layer is formed) on the support layer according to the second example when the well layer is observed with a scanning electron microscope.

Referring to FIGS. 14 and 15 together, in the second example, a well array filter was manufactured under the condition in which the well inter-bottom width (4.6 µm) is smaller than the well wall width (8.8 µm). In the second example, the well bottom body width was 55.6 µm, and the well opening width was 52.5 µm.

### <Observation result of rear surface of well array filter>

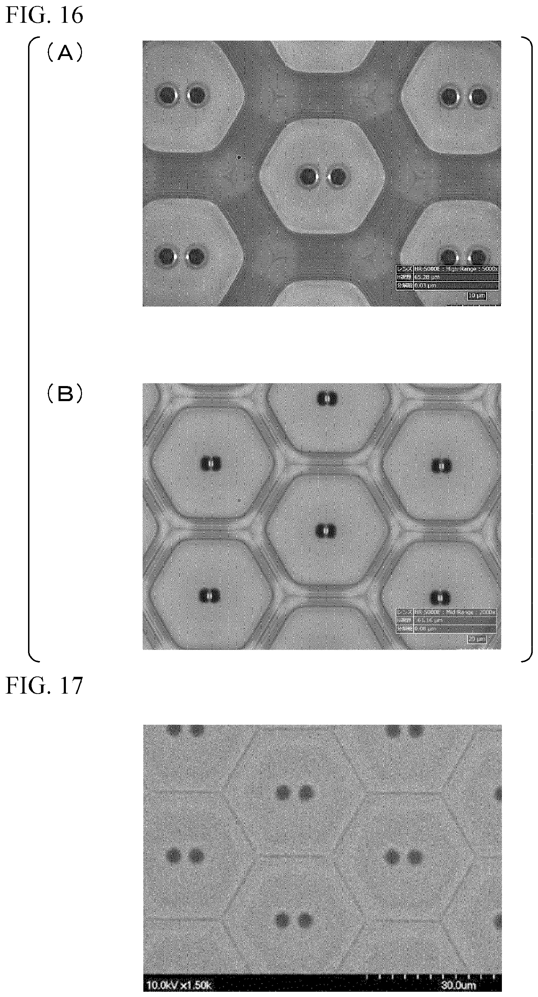

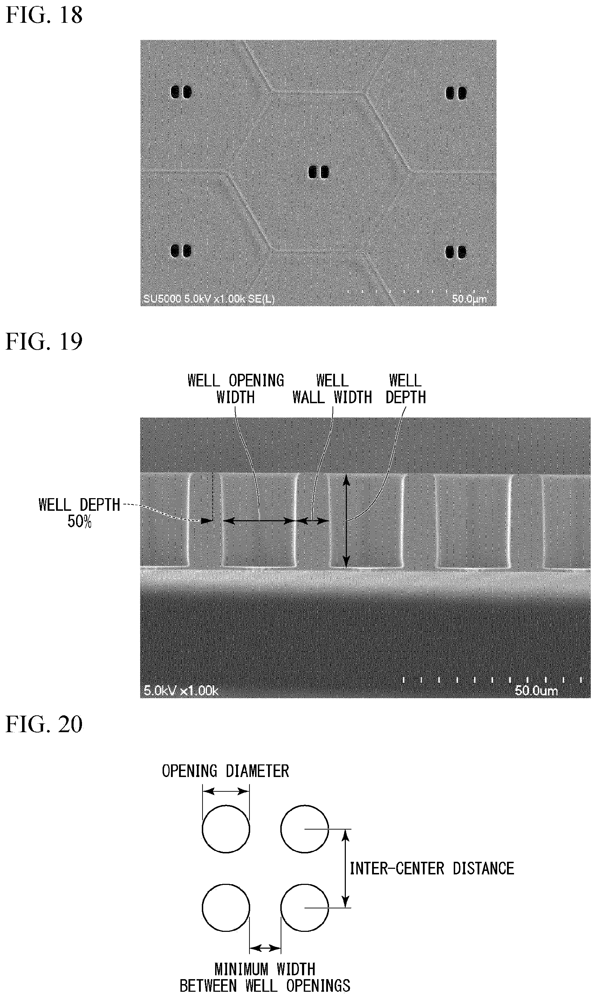

FIG. 16 is a diagram illustrating a rear surface of a well array filter according to an example (corresponding to a well opening width of 20 µm and a well bottom body width of 25 µm) when the rear surface is observed with an optical microscope (specifically a digital microscope HRX-01 made by HirRox CO., Ltd.: imaged using a coaxial episcopic illumination). FIG. 16(A) illustrates observation at 5000 magnification, and FIG. 16(B) illustrates observation at 2000 magnification. FIG. 17 is a diagram illustrating a rear surface of a well array filter according to an example (corresponding to a well opening width of 20 µm) when the rear surface is observed with a scanning electron microscope (specifically a S-4700 electric field emission type scanning electron microscope made by Hitachi High-Tech Corporation: observation with an acceleration voltage of 10 kV and at 1500 magnifications). FIG. 18 is a diagram illustrating a rear surface of a well array filter according to an example (corresponding to a well opening width of 50 µm) when the rear surface is observed with a scanning electron microscope (specifically a SU-5000 electric field emission type scanning electron microscope made by Hitachi High-Tech Corporation: observation with an acceleration voltage of 5 kV and at 1000 magnifications).

Referring to FIGS. 16 to 18 together, it was ascertained that a line appeared in a part along the outer shape of the well bottoms in the well array filters according to the examples when the rear surface of the well array filter (corresponding to the bottom surface) was observed with an optical microscope or a scanning electron microscope.

As described above, in the well array filters according to the examples, a line appeared in a part along the outer shape of the well bottoms when the rear surface of the well array filter (corresponding to the bottom surface) was observed with an optical microscope or a scanning electron microscope.

With this configuration, since the structure according to the present invention can be identified when a line appears, it is possible to contribute to facilitation of infringement identification.

### <Examples in which independent well array filter with high aperture ratio is manufactured>

FIG. 19 is a diagram illustrating dimensions along with a sectional view of the well array filters according to the examples when observed with a scanning electron microscope. Table 1 is a table showing dimensions along with conditions for manufacturing the well array filters according to the examples.

**Table 1**

| | Shape | Well opening width /µm | Well wall width /µm | Well opening ratio /% | Well density /cm⁻² | Well depth /µm | Well depth / well wall width ratio |
|---|---|---|---|---|---|---|---|
| Example 1 | Hex20-25 | 20.34 | 9.62 | 46.1 | 128643 | 26.39 | 2.74 |
| Example 2 | Hex20-25 | 25.1 | 5.46 | 67.5 | 123641 | 23.51 | 4.31 |
| Example 3 | Hex20-50 | 20.1 | 10.1 | 44.3 | 126606 | 49.2 | 4.87 |
| Example 4 | Hex20-50 | 19.3 | 10.6 | 41.7 | 129160 | 47.1 | 4.44 |
| Example 5 | Hex20-50 | 22.1 | 7 | 57.7 | 136359 | 51.2 | 7.31 |
| Example 6 | Hex50-50 | 51.3 | 9 | 72.4 | 31757 | 49.9 | 5.54 |
| Example 7 | Hex50-50 | 58.2 | 2.8 | 91.0 | 31032 | 35.5 | 12.68 |
| Example 8 | Hex50-50 (bridged tile) | 50.8 | 8.5 | 73.4 | 32837 | 53.8 | 6.33 |

Referring to FIG. 19 and Table 1 together, the well opening width and the well wall width are values measured at a position of 50% of the well depth in the image obtained when cross-sections of the well array filters according to the examples were observed with the scanning electron microscope.

In Examples 1 to 5, a pattern was manufactured while changing an amount of exposure at the time of formation of a well layer using reticle 1 (an example of a photomask). Reticle 1 was a mask for manufacturing a well array filter including well openings which are hexagonal in a plan view and was designed such that the well bottom body width was 27.5 µm, the well inter-bottom width was 2.5 µm, the well opening width was 20 µm, and the well wall width was 10 µm.

In Examples 6 and 7, a pattern was manufactured while changing an amount of exposure at the time of formation of a well layer using reticle 2 (an example of a photomask). Reticle 2 was a mask for manufacturing a well array filter including well openings which are hexagonal in a plan view and was designed such that the well bottom body width was 56 µm, the well inter-bottom width was 4 µm, the well opening width was 52 µm, and the well wall width was 8 µm.

In Example 8, a pattern was manufactured while changing an amount of exposure at the time of formation of a well layer using reticle 3 (an example of a photomask). Reticle 3 was a mask for manufacturing a well array filter including well openings which are hexagonal in a plan view and was designed such that the well bottom body width was 56 µm, the well inter-bottom width was 4 µm, the connecting portion width was 10 µm, the well opening width was 52 µm, and the well wall width was 8 µm.

FIG. 20 is a diagram illustrating dimensions along with a top view of a well. Table 2 is a table for comparison between the prior patent (the related art) and the examples.

**Table 2**

| Opening shape | Number | Material | Well opening width /µm (opening diameter) | Inter-center distance /µm | Minimu m width between well openings /µm | Well opening ratio /% | Well density /cm⁻² | Well depth /µm | Well depth / well wall width ratio |
|---|---|---|---|---|---|---|---|---|---|
| Circular | Japanese Unexamined Patent Application, First Publication No. 2016-010761 | Resist | 10 | 50 | 40 | 3.1 | 40000 | 10 | 0.25 |
| | Japanese Unexamined Patent Application, First Publication No. 2016-182553 | Resist | 10 | 14 | 4 | 40.1 | 520204 | 10 | 2.5 |
| | Japanese Unexamined Patent Application, First Publication No. 2004-173681 | PDMS | 10 | 40 | 30 | 4.9 | 62500 | 36 | 1.2 |
| | | | 10 | 25 | 15 | 12.6 | 160000 | 12 | 0.8 |
| | US9638636 B2 | Si | 100 | 150 | 50 | 34.9 | 4444 | 380 | 7.6 |
| | Japanese Patent Publication No. 2020-533567 | Photo-curable resin | 20 | 80 | 60 | 4.9 | 15625 | 20 | 0.3 |
| | WO2015-012315 | Pd-Ni | 30 | 34 | 4 | 61.1 | 86505 | 10 | 2.5 |
| Hexago nal | US10466160 B2 | ? | 25.98 | - | 3 | 80.4 | 137490 | 30 | 10 |
| | Example 1 (Hex20-25) | Resist | 20.34 | - | 9.62 | 46.1 | 128643 | 26.39 | 2.74 |
| | Example 2 (Hex20-25) | Resist | 25.1 | - | 5.46 | 67.5 | 123641 | 23.51 | 4.31 |
| | Example 3 (Hex20-50) | Resist | 20.1 | - | 10.1 | 44.3 | 126606 | 49.2 | 4.87 |
| | Example 4 (Hex20-50) | Resist | 19.3 | - | 10.6 | 41.7 | 129160 | 47.1 | 4.44 |
| | Example 5 (Hex20-50) | Resist | 22.1 | - | 7 | 57.7 | 136359 | 51.2 | 7.31 |
| | Example 6 (Hex50-50) | Resist | 51.3 | - | 9 | 72.4 | 31757 | 49.9 | 5.54 |
| | Example 7 (Hex50-50) | Resist | 58.2 | - | 2.8 | 91.0 | 31032 | 35.5 | 12.68 |
| | Example 8 (Hex50-50) | Resist | 50.8 | - | 8.5 | 73.4 | 32837 | 53.8 | 6.33 |

Referring to FIG. 20 and Table 2 together, the well opening width was set to the opening diameter (the diameter of the well opening) when the well opening has a circular shape in a plan view in the prior patent. The minimum width between the well openings was set to a minimum width of a wall portion defining two neighboring well openings in a plan view when the well opening had a circular shape in a plan view in the prior patent. The inter-center distance was set to a distance between the centers of two neighboring well openings in a plan view when the well openings had a circular shape in a plan view in the prior patent.

In the examples, a well array filter obtained by cooling and then cutting a patterned wafer with liquid nitrogen and then immersing the resultant in p-menthane was observed with a scanning electron microscope. The size in a plan view of the obtained well array filter was set to 21 mm×21 mm.

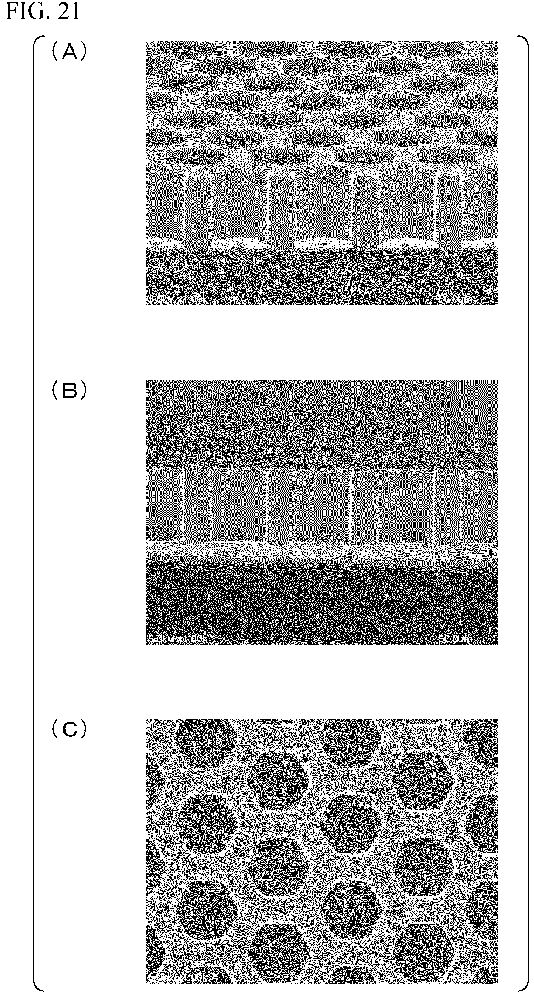

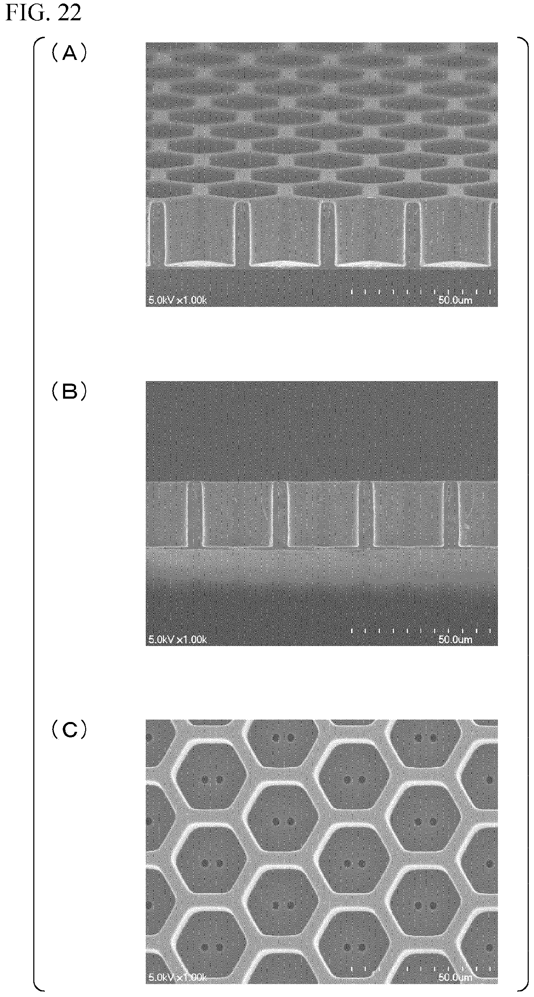

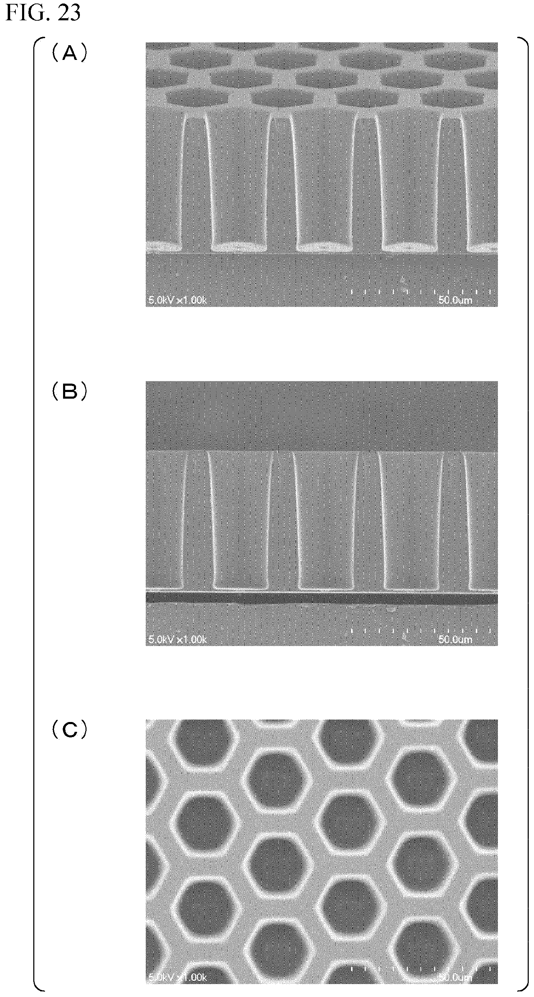

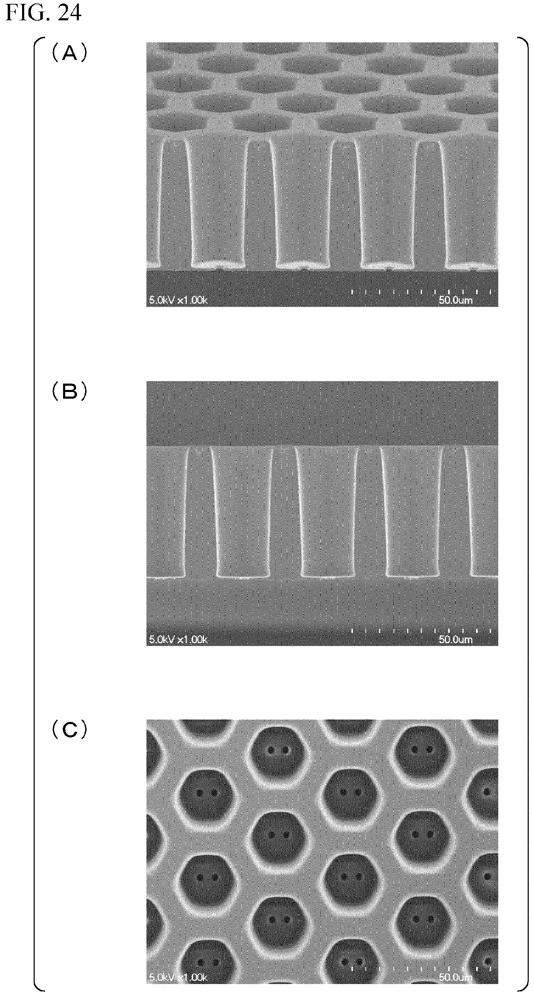

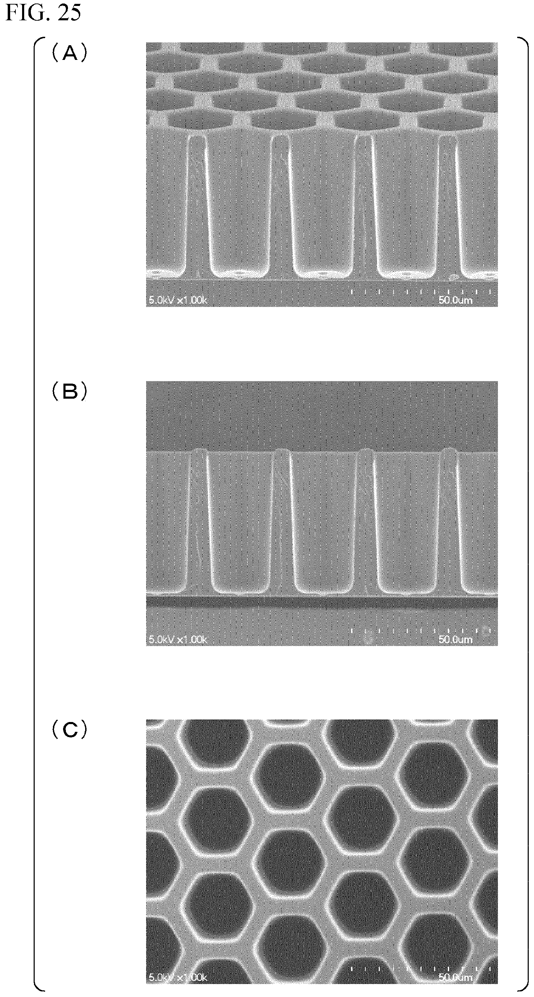

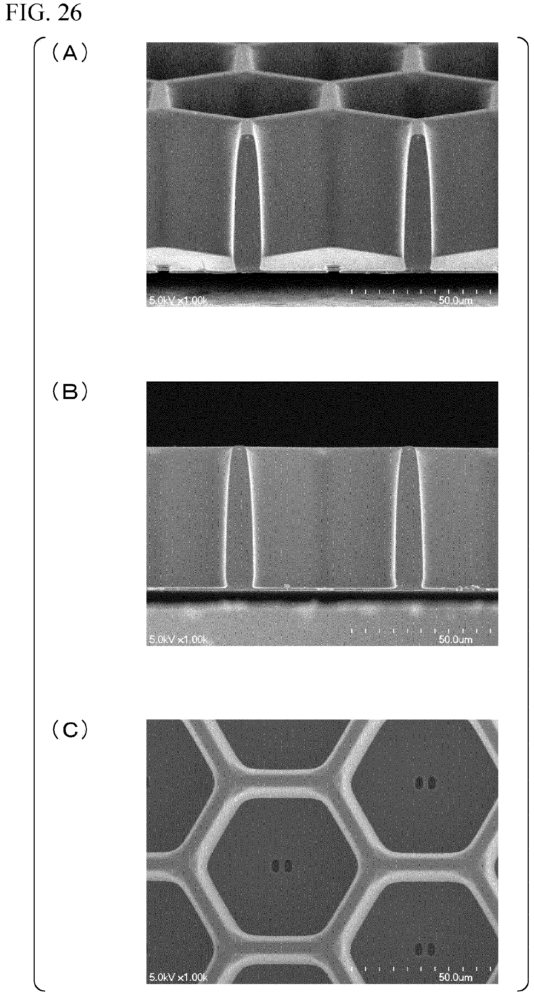

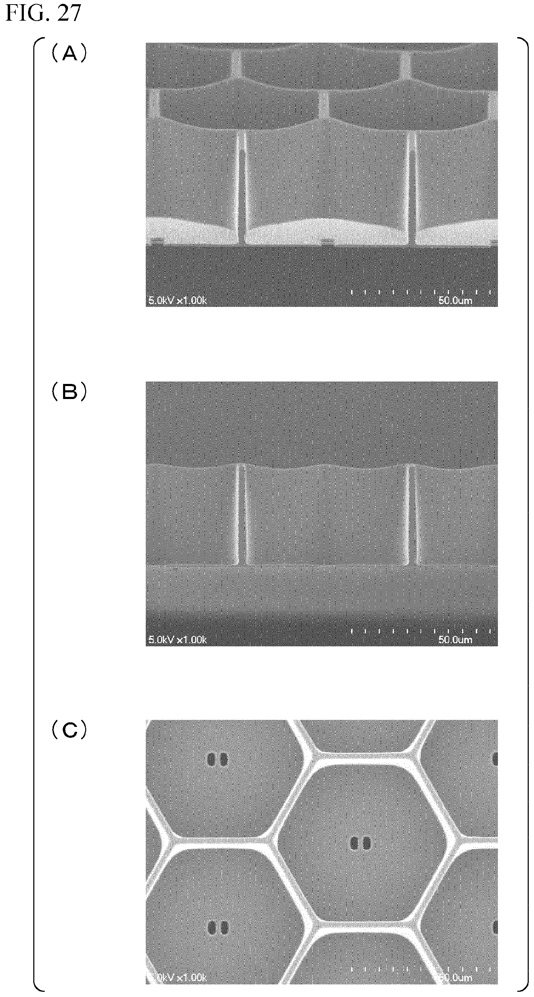

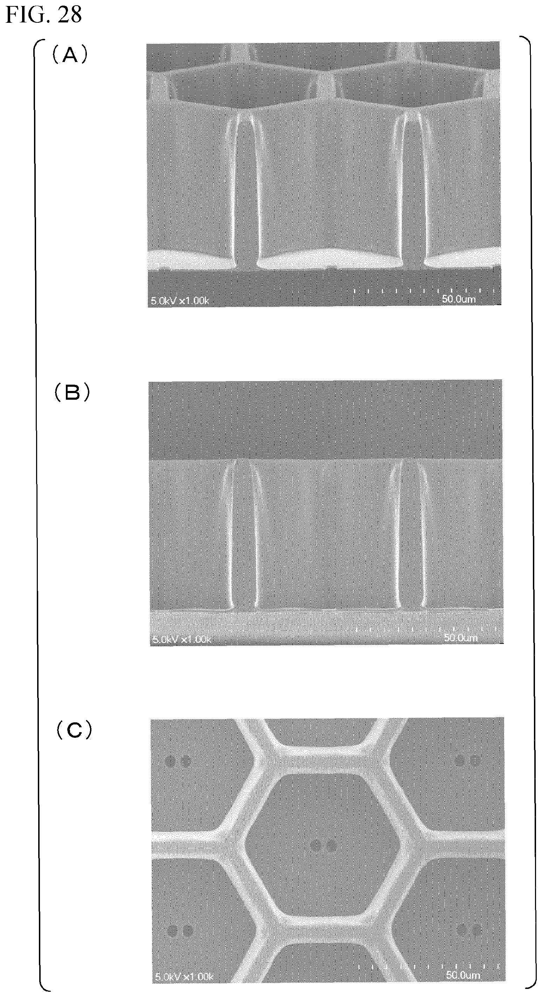

FIG. 21 is a diagram illustrating a well array filter according to Example 1 when the well array filter was observed with a scanning electron microscope. FIG. 21(A) is a perspective view, FIG. 21(B) is a sectional view, and FIG. 21(C) is a top view. FIG. 22 is a diagram illustrating a well array filter according to Example 2 when the well array filter was observed with a scanning electron microscope. FIG. 22(A) is a perspective view, FIG. 22(B) is a sectional view, and FIG. 22(C) is a top view. FIG. 23 is a diagram illustrating a well array filter according to Example 3 when the well array filter was observed with a scanning electron microscope. FIG. 23(A) is a perspective view, FIG. 23(B) is a sectional view, and FIG. 23(C) is a top view. FIG. 24 is a diagram illustrating a well array filter according to Example 4 when the well array filter was observed with a scanning electron microscope. FIG. 24(A) is a perspective view, FIG. 24(B) is a sectional view, and FIG. 24(C) is a top view. FIG. 25 is a diagram illustrating a well array filter according to Example 5 when the well array filter was observed with a scanning electron microscope. FIG. 25(A) is a perspective view, FIG. 25(B) is a sectional view, and FIG. 25(C) is a top view. FIG. 26 is a diagram illustrating a well array filter according to Example 6 when the well array filter was observed with a scanning electron microscope. FIG. 26(A) is a perspective view, FIG. 26(B) is a sectional view, and FIG. 26(C) is a top view. FIG. 27 is a diagram illustrating a well array filter according to Example 7 when the well array filter was observed with a scanning electron microscope. FIG. 27(A) is a perspective view, FIG. 27(B) is a sectional view, and FIG. 27(C) is a top view. FIG. 28 is a diagram illustrating a well array filter according to Example 8 when the well array filter was observed with a scanning electron microscope. FIG. 28(A) is a perspective view, FIG. 28(B) is a sectional view, and FIG. 28(C) is a top view.

Referring to FIGS. 21 to 28 together, it was ascertained in any condition of Examples 1 to 8 that an independent well array filter was obtained.

### <Example in which well array filter according to comparative example including continuous support layer was manufactured>

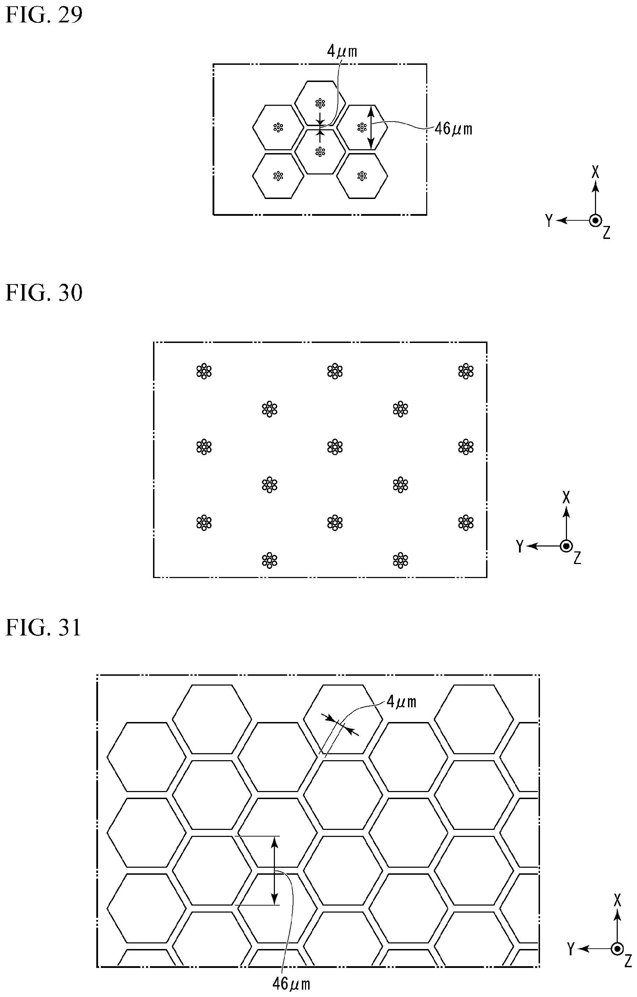

FIG. 29 is a top view of a well array filter according to a comparative example. FIG. 30 is a top view of a support layer according to a comparative example. FIG. 31 is a top view of a well layer according to a comparative example.

Referring to FIGS. 29 to 31 together, a well array filter according to a comparative example was manufactured by superimposing a well layer on a continuous support layer. In the comparative example, the well array filter was manufactured such that the thickness of the continuous support layer was 1 µm, the thickness of the well layer was 50 µm, the well bottom body width was 46 µm, and the well inter-bottom width was 4 µm.

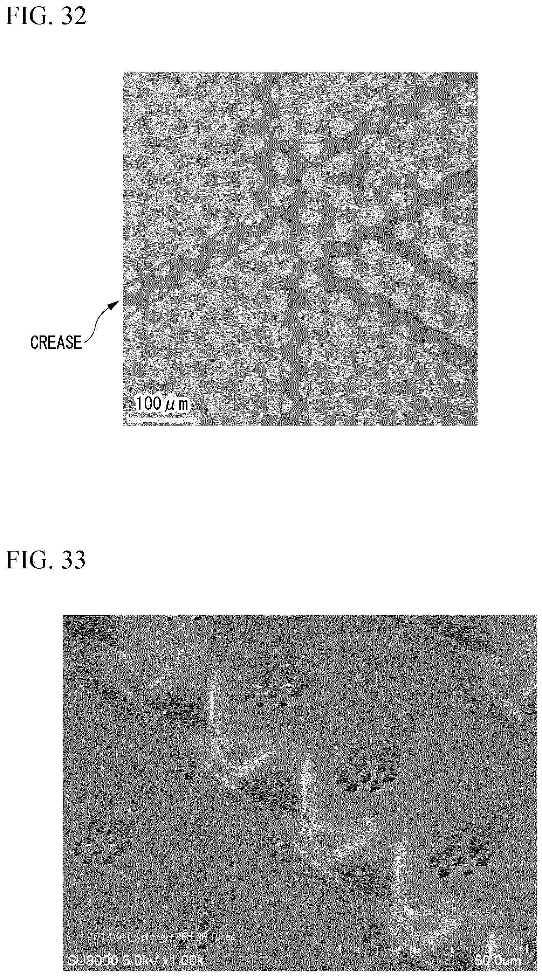

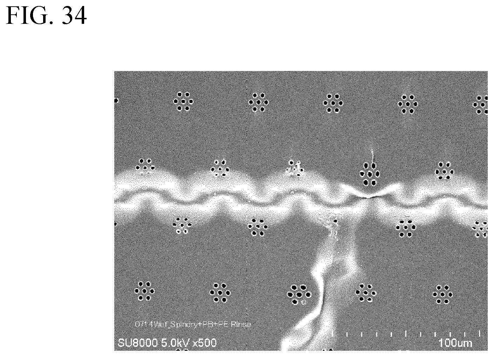

FIG. 32 is a diagram illustrating a top surface of a well array filter according to a comparative example when the well array filter is observed with an optical microscope. FIG. 33 is a diagram illustrating a rear surface of a well array filter according to a comparative example when the well array filter is observed with a scanning electron microscope. FIG. 34 is a diagram illustrating a rear surface of a well array filter according to a comparative example when the well array filter is observed with a scanning electron microscope.

Referring to FIGS. 32 to 34 together, in the comparative example, it was ascertained that creases (a crease-like defect) appeared when the top surface or the rear surface of the well array filter was observed with an optical microscope or a scanning electron microscope. The reason is estimated to be that the well layer and the continuous support layer were mechanically separated from the sacrificial film and lifted upward by a stress acting on the continuous support layer due to curing shrinkage of the well layer and a stress due to curing shrinkage of the continuous support layer.

As described above, according to these examples, it was ascertained that a structure in which the well opening ratio was higher than that in the prior patent and the well depth was larger than that could be manufactured. Accordingly, according to these examples, it could be seen that it is possible to minimize cells or particles not entering the well openings and left on the wall and to arrange the cells or particles with a high density.

### INDUSTRIAL APPLICABILITY

With the well array filter, the well array device, and the well array filter manufacturing method according to the present invention, it is possible to provide an independent well array filter with a high aperture ratio.

### REFERENCE SIGNS LIST

1 Well array filter
3 Subject
10 Well layer
11 Well opening
12 Well wall
20 Support layer
21 Well bottom
22 Subject placement surface
23 Through-hole
30 Substrate
31 Sacrificial film
327 Well bottom structure
A Well depth (depth of well layer)
B Well wall width (minimum width of partition wall portion between neighboring wells in well layer)
S Well inter-bottom width (minimum width between divided well bottoms)
T Thickness of support layer

## Claims

1. A well array filter comprising:
a through-hole of a size through which a subject including at least one of a cell, a particle, and a liquid droplet does not pass in a well bottom;
a well layer including a plurality of well openings of a size through which the subject is able to pass; and
a support layer supporting the well layer,
wherein the support layer includes a plurality of well bottoms corresponding to the well openings, and
wherein at least a part of the support layer between a plurality of neighboring well bottoms is divided.

2. The well array filter according to claim 1, wherein the support layer is completely divided for each well bottom.

3. The well array filter according to claim 1, wherein the support layer includes a plurality of well bottom structures in which a plurality of well bottoms are connected together and the support layer is divided for each well bottom structure.

4. The well array filter according to any one of claims 1 to 3, wherein the well array filter is formed of a photo-curable resin.

5. The well array filter according to any one of claims 1 to 3, wherein an aperture ratio of the well openings is equal to or greater than 40%,
wherein a ratio A/B of a depth A of the well layer to a minimum width B of a partition wall portion between neighboring wells in the well layer is equal to or greater than 2, and
wherein a thickness of the support layer is equal to or less than 5 µm.

6. The well array filter according to any one of claims 1 to 3, wherein the support layer includes the through-holes communicating with the well openings.

7. The well array filter according to any one of claims 1 to 3, wherein each well opening has a polygonal shape when seen in a thickness direction of the well layer.

8. The well array filter according to any one of claims 1 to 3, wherein the well layer includes a well wall partitioning the well openings, and
wherein the plurality of well bottoms are divided in a part overlapping a central position in a width direction of the well wall when seen in a thickness direction of the well layer.

9. The well array filter according to any one of claims 1 to 3, wherein a minimum width between the divided well bottoms when seen in a thickness direction of the support layer is larger than a thickness of the support layer.

10. The well array filter according to any one of claims 1 to 3, further comprising a subject placement surface on which the subject is placed,
wherein the subject placement surface is covered with a non-cell-adhesive material.

11. The well array filter according to any one of claims 1 to 3, wherein a line appears in a part along an outer shape of the well bottoms when a rear surface of the well array filter is observed with an optical microscope or a scanning electron microscope.

12. A well array device for accommodating and arranging the subject, the well array device comprising the well array filter according to any one of claims 1 to 3.

13. A well array filter manufacturing method of manufacturing the well array filter according to any one of claims 1 to 3, the well array filter manufacturing method comprising:
a first step of forming a sacrificial film which is insoluble in a solvent dissolving materials of the support layer and the well layer on a substrate;
a second step of forming the support layer and the well layer on the sacrificial film after the first step; and
a third step of dissolving the sacrificial film and separating a structure including the support layer and the well layer from the substrate after the second step.
